# EUROPEAN PATENT SPECIFICATION

(11) **EP 1 390 000 B1**
(45) Date of publication and mention of the grant of the patent: **02.11.2006**
(21) Application number: 02720044.3
(22) Date of filing: 30.04.2002
(51) Int. Cl.: A61K 8/44, A61Q 5/00, A61Q 5/08

(54) **USE OF BETAINE IN HAIR CARE**
VERWENDUNG VON BETAIN IN DER HAARPFLEGE
UTILISATION DE LA BETAINE POUR LE SOIN CAPILLAIRE

(30) Priority: 02.05.2001 FI 20010911; 04.02.2002 FI 20020213
(43) Date of publication of application: 25.02.2004
(73) Proprietor: Finnfeeds Finland OY, 02460 Kantvik (FI)
(72) Inventor: JUTILA, Kirsti, FIN-02170 ESPOO (FI)
(74) Representative: HOFFMANN EITLE
(86) International application number: PCT/FI2002/000367
(87) International publication number: WO 2002/087526

(56) References cited:
- WO-A1-91/18588
- US-A- 4 752 467
- PATENT ABSTRACTS OF JAPAN & JP 06 293 621 A (ASAHI CHEM IND CO LTD) 21 October 1994
- PATENT ABSTRACTS OF JAPAN & JP 08 217 651 A (SUNSTAR INC) 27 August 1996
- L. RIGANO ET AL.: 'Benefits of trimethylglycine (Betaine) in personal-care formulations' COSMETICS & TOILETRIES vol. 115, no. 12, December 2000, pages 47 - 52, AND 54, XP002954567

## Description

The present invention relates to use of betaine for enhancing and/or maintaining the elasticity of hair. More particularly, it relates to use of betaine for enhancing and/or maintaining the elasticity of chemically treated hair.

### Background of the invention

Hair is continuously subjected to various conditions that may dry, weaken and/or damage it. These include for example shampooing, rinsing, combing, drying, styling, permanent waving, bleaching and dyeing. These processes reduce the strength and elasticity of the hair. Thus, the hair is often dry, lustreless or brittleness.

U.S. patent No. 4,752,467 describes a hair treatment composition comprising betaine, and at least one aliphatic organic acid for strengthening and conditioning human hair. Japanese patent publication No. 6 293 621 discloses a perming solution containing betaine, which is said to have excellent hair protecting action. Further, Japanese patent publication No. 8217651 discloses an acidic hair dye composition comprising betaine. This composition is described to show excellent hair-dyeing ability, penetrating properties and dyed color fastness. In addition to these publications, Rigano, L., et al (2000) *Cosmetics & Toiletries magazine* Vol. 115, No.12, p 47-54 describes generally the benefits of betaine in personal-care formulations in the cosmetic's field.

However, it does not appear from any of the above-mentioned references that betaine enhances and/or maintains the elasticity of chemically treated hair.

### Summary of the invention

The present invention relates to use of betaine for enhancing and/or maintaining the elasticity of chemically treated hair. Further, the present invention relates to the use of betaine for enhancing and/or maintaining the elasticity of chemically treated hair before, simultaneously with or after further colouring it with a desired hair dye.

In addition, the present invention relates to a method for enhancing and/or maintaining the elasticity of chemically treated hair comprising applying betaine into hair. Further, the present invention relates to a method for enhancing and/or maintaining the elasticity of chemically treated hair comprising applying betaine to hair without subsequent rinsing.

The present invention relates also to a method for enhancing and/or maintaining the elasticity of chemically treated hair comprising applying betaine before, simultaneously with or after further colouring it with a desired hair dye.

### Detailed description of the invention

The applicant has now discovered that betaine enhances the elasticity and/or the tensile strength of hair, especially chemically treated hair. An aqueous betaine solution was found to strengthen the hair in both elastic and yield regions when applied to bleached hair. In addition, betaine was found to extend the gloss and brightness of the hair. It also increased the body of hair.

In the present invention betaine refers to trimethylglycine, a compound having the formula

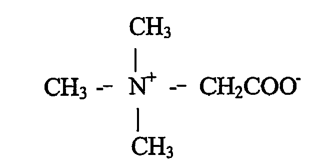

The compound is also called trimethylammonioacetate, 1-carboxy-N,N,N-trimethyhnethaneaminium, inner salt, or glycine betaine. In its pure form, betaine is a white, crystalline compound, which is readily soluble in water and lower alcohols, such as methanol and ethanol. Owing to its zwitterion (amphoteric) structure, betaine is capable of neutralizing both acid and alkaline solutions.

Betaine is commercially available from Finnfeeds Finland Ltd. In the present invention betaine can be used for example as an anhydride, a monohydrate or a salt.

In the present invention the term elasticity refers to the flexibility of hair and/or to the tensile strength of hair. Further, in the present invention chemical treatment refers to bleaching or dyeing, and chemically treated hair refers to bleached or dyed hair.

The chemical processes related to bleaching or dyeing hair are very hard causing reduction in the elastic modulus and elastic gradient of the hair compared to untreated hair. Especially, when dyeing dark hair, such as dark European type of hair or specifically Oriental and Ethnic hair, with a colour lighter that the original one, the original colour needs to be removed, for example by bleaching, before colouring it with the desired dye. During these processes the hair is subject to considerable chemical damage, which is seen as a reduction in the elastic modulus and elastic gradient of the hair.

In the present invention, betaine was found to have a pronounced effect on the elasticity of chemically treated hair. The application of betaine solution to chemically treated European type of hair and Oriental hair without subsequent rinsing had a significant effect compared to a water placebo. The use of betaine solution to simulate the action of a rinse-off conditioner product on chemically treated hair had also a significant effect compared to a water placebo on the Oriental hair.

According to the present invention, betaine may be applied to hair before, simultaneously with or after the chemical treatment of the hair. In one embodiment of the present invention betaine is used for enhancing and/or maintaining the elasticity of chemically treated hair before, simultaneously with, or after further colouring the hair with a desired hair dye.

In the present invention betaine could be applied into hair in the form of, such as but not limited to, an aqueous solution or a conventional conditioning or shampooing composition. A conventional hair care composition may be in the form of a lotion, mousse, spray, gel or hair mask. It may be a rinse-off or leave-on formulation. In a preferred embodiment of the present invention betaine is formulated into a leave-on conditioning formulation. Further, betaine could be incorporated into a bleaching or colourant formulation. In addition to enhancing and/or maintaining the elasticity of hair, betaine also protects hair against further damage when, for example, the bleached hair is subsequently dyed. Betaine also increases the colour intension of the hair dye. Betaine also extends the gloss and brightness of the hair and increases the body of the hair.

The amount of betaine to be used according to the present invention depends for example on the condition of the hair, the origin of the hair or the formulation of the hair care product. The amount of betaine may vary from about 0.1 % to about 50 % by weight, preferably from about 0.5 % to about 15% by weight.

The invention will be described in more detail by means of the following examples. The examples are provided only in order to illustrate the invention and they should not be construed to restrict the scope of the invention in any way.

### EXAMPLES

The effect of betaine on the elasticity of hair was studied by measuring the tensile strength of hair.

The method used in this study broadly follows the guidelines laid down in BS EN ISO 5079:1996 for the tensile testing of fibres. The measurements were done with a Dia-Stron MTT170 tensile test machine. For each measurement, 50 hairs were taken randomly from the tress. Each hair was individually mounted in the tensile jig and pulled, under controlled conditions, at a rate of 20 mm/min, until breakage occurs. The jig operated under computer control, and for each hair, records applied load against extension. Also the diameter of each hair was measured, to a resolution of 1 micron, using a digital micrometer. Using the measured hair diameters and a fixed gauge length, the data was converted to stress (load/unit area) against strain (% extension).

### Example 1

Six tresses, three of European type of hair and three of Oriental hair, were subjected to a bleaching pre-treatment. The tresses were treated twice in succession, in order to provide damaged samples for this study. The process was carried out with the tresses of each type as a group, such that they each received exactly the same extent of processing. As the hair of each group was obtained from the same source, each group may be considered to comprise three identical stresses. In order to standardise their condition prior to commencement of the study, all six tresses were washed in a 15% active solution of sodium laureth sulphate (SLES) and left to dry naturally.

Further, in order to provide a baseline for the evaluation, hairs were taken from one tress in each group, and were measured in this untreated condition. The remaining four tresses were treated following the protocol detailed below, and hairs taken from them were then measured in the same manner.

Two tresses, one of European type of hair and one of Oriental hair, were wetted and 1 g of SLES solution was massaged through the hair of each tress for 60 seconds to simulate usage of a shampoo. The tresses were then rinsed for 60 seconds, excess water being removed by squeezing the hair through thumb and forefinger. 1 g of 5% aqueous betaine solution was then massaged through the hair of each tress for 30 seconds, and then left on the hair for 15 minutes to simulate an intensive conditioning treatment before being rinsed for 60 seconds. Excess water was again removed by squeezing the hair through thumb and forefinger, following which the tresses were combed, and left to dry naturally. The procedure described above was then repeated for a total of five full cycles, the tresses being re-washed with SLES solution, rinsed and retreated with betaine solution, to simulate repeated application of a shampoo and a conditioner.

Two further stresses, again one of European type of hair and one of Oriental hair, were treated, following the same washing procedure, but with water applied in place of the betaine solution. Again five full cycles were carried out, in order to ensure that the hair in these tresses received the same amount of mechanical abrasion, and was able to serve as a placebo control.

The results (mean values) are presented in Tables 1 and 2 for the European type of hair and the Oriental hair, respectively.

**Table 1. European type of hair**

| Tress | Elastic modulus MPa | Elastic gradient gmf/mm | Plateau stress (gmf/sq micron) | Stress at 15% (gmf/sq micron) | Stress at 25% (gmf/sq micron) | Break stress (gmf/sq micron) |
|---|---|---|---|---|---|---|
| Treated with betaine | 2.230 | 18.4 | 1.40E-002 | 1.43E-002 | 1.78E-002 | 3.24E-002 |
| Treated with water | 2.600 | 19.7 | 1.43E-002 | 1.49E-002 | 1.84E-002 | 3.48E-002 |
| Untreated base tress | 3.320 | 29.7 | 1.28E-002 | 1.33E-002 | 1.65E-002 | 3.11E-002 |

The results show that for European type of hair both elastic modulus and elastic gradient were significantly reduced after the treatment with either water or betaine solution. These results indicate that the considerable degree of mechanical manipulation involved in the washing/combing/drying process had damaged the hair even further, reducing its elasticity. Plateau stress, stress at 15%, and stress at 25% were all significantly higher after treatment with either water or betaine solution, compared to the untreated baseline hair.

**Table 2. Oriental hair**

| Tress | Elastic modulus MPa | Elastic gradient gmf/mm | Plateau stress (gmf/sq micron) | Stress at 15% (gmf/sq micron) | Stress at 25% (gmf/sq micron) | Break stress (gmf/sq micron) |
|---|---|---|---|---|---|---|
| Treated with betaine | 4.660 | 58.2 | 1.09E-002 | 1.46E- 002 | 1.46E-002 | 2.86E-002 |
| Treated with water | 2.420 | 31.7 | 1.01E-002 | 1.05E-002 | 1.33E-002 | 2.70E-002 |
| Untreated base tress | 3.020 | 40.0 | 1.05E-002 | 1.08E-002 | 1.36E-002 | 2.72E-002 |

The results show that as for the European type of hair, both elastic modulus and elastic gradient of the Oriental hair were significantly reduced after the treatment with water. This indicates that that the considerable degree of mechanical manipulation involved in the washing /combing/drying process damaged the hair even further, reducing its elasticity. However, following treatment with betaine solution, figures for these parameters were both significantly higher. This result indicates that the betaine treatment has not only overcome the manipulation damage, but has further improved the condition of the hair in this elastic region.

Comparing the tress treated with betaine solution to that treated with water, the parameters of elastic modulus, elastic gradient, stress at 15% and stress at 25% showed a significant difference at p<0.05. In addition the values for plateau stress were significant at p=0.06. Accordingly, betaine treatment helped to strengthen the hair in both the elastic and yield regions.

### Example 2

Eight tresses, five of European type of hair and three of Oriental hair, were subjected to a bleaching pre-treatment in order to provide damaged samples for the study. The tresses of European type of hair were bleached once while the Oriental ones were bleached twice. This process was carried out with the tresses of each type as a group, such that they each received exactly the same extent of processing. As the hair of each group was obtained from the same source, each group may reasonably be considered to comprise identical tresses. In order to standardise their condition prior to commencement of the trial, all eight stresses were washed in a 15% active solution of sodium laureth sulphate (SLES) and left to dry naturally.

In order to provide a baseline for the evaluation, hairs were taken from one tress in each group, and were measured in this untreated condition. The remaining tresses were treated following the protocol detailed below, and hairs taken from them were then measured in the same manner.

One tress of European type of hair was wetted and 1 g of SLES solution was massaged through the hair for 60 seconds to simulate usage of a shampoo. The tress was then rinsed for 60 seconds, with excess water being removed by squeezing the hair through thumb and forefinger. 1 g of 5% aqueous betaine solution was then massaged through the hair for 30 seconds, and was then left on the hair for 15 minutes to simulate an intensive conditioning treatment before being rinsed for 60 seconds. Excess water was again removed by squeezing the hair through thumb and forefinger, following which the tress was combed, and left to dry naturally. The procedure described above was then repeated for a total of five full cycles, one per day, the tresses being re-washed with SLES solution, rinsed and retreated with betaine solution, to simulate repeated application of a shampoo and a conditioner.

A further stress of European type of hair was treated, following the same washing procedure, but with water applied in place of the betaine solution, as a negative control. Again five full cycles were carried out, in order to ensure that the hair in the tress received the same amount of mechanical abrasion, and was able to serve as a placebo control.

Two further tresses, one of European type of hair and one of Oriental hair, were then subjected to the same washing and betaine treatment procedure described above, but after 1 g of the betaine solution had been massaged through the hair for 30 seconds, the hair was combed once and left to dry without rinsing.

Two further tresses, again one of European type of hair and one of Oriental hair, were treated following the same washing procedure but with water applied in place of the betaine solution, as a negative control, in order to ensure that the hair in these tresses received the same amount of mechanical abrasion, and were able to serve as a placebo control.

The results (mean values) for the European type of hair treated for 5 cycles of 15 min application are presented in Tables 3a and 3b.

**Table 3a**

| Tress | Elastic modulus MPa | Elastic gradient gmf/mm | Plateau stress (gmf/sq micron) | Stress at 15% (gmf/sq micron) | Stress at 25% (gmf/sq micron) | Break stress (gmf/sq micron) |
|---|---|---|---|---|---|---|
| Treated with betaine | 1.990 | 28.9 | 9.76E-003 | 9.94E-003 | 1.24E-002 | 2.69E-002 |
| Treated with water | 2.050 | 28.9 | 9.97E-003 | 1.03E-002 | 1.27E-002 | 2.63E-002 |
| Untreated base tress | 2.020 | 28.4 | 9.05E-003 | 9.12E-003 | 1.15E-002 | 2.62E-002 |

**Table 3b**

| Tress | Elastic extension (%) | Work @ 15% (joules) | Work @ 25% (joules) | Yield extension (%) | Break extension (%) | Total work (joules) |
|---|---|---|---|---|---|---|
| Treated with betaine | 3.24 | 1.38E-003 | 2.80E-003 | 27.1 | 51.1 | 9.31E-003 |
| Treated with water | 3.2 | 1.38E-003 | 2.80E-003 | 27.3 | 51.6 | 9.60E-003 |
| Untreated base tress | 2.88 | 1.26E-003 | 2.53E-003 | 27.4 | 52 | 8.90E-003 |

The results show that for European type of hair, treatment with betaine and treatment with water have both the same effect compared to the untreated tress. There were no significant difference between these treatments and, in both cases it was mainly the action of a liquid treatment, regardless of its chemical content, which helped to strengthen the hair. This is very similar result to that obtained in Example 1. However, there the hair was double bleached to a very poor condition before the treatment. In this example the was bleached only once, which improved the base condition of the hair.

The results (mean values) for the European type of hair with the treatment not rinsed from hair are presented in Table 4a and 4b.

**Table 4a**

| Tress | Elastic modulus MPa | Elastic gradient gmf/mm | Plateau stress (gmf/sq micron) | Stress at 15% (gmf/sq micron) | Stress at 25% (gmf/sq micron) | Break stress (gmf/sq micron) |
|---|---|---|---|---|---|---|
| Treated with betaine | 1.910 | 28.1 | 9.34E-003 | 9.40E-003 | 1.17E-002 | 2.67E-002 |
| Treated with water | 1.940 | 28.5 | 8.34E-003 | 8.20E-003 | 1.05E-002 | 2.61E-002 |
| Untreated base tress | 2.020 | 28.4 | 9.05E-003 | 9.12E-003 | 1.15E-002 | 2.62E-002 |

**Table 4b**

| Tress | Elastic extension (%) | Work @ 15% (joules) | Work @ 25% (joules) | Yield extension (%) | Break extension (%) | Total work (joules) |
|---|---|---|---|---|---|---|
| Treated with betaine | 3.25 | 1.35E-003 | 2.72E-003 | 27.8 | 52.5 | 9.57E-003 |
| Treated with water | 2.89 | 1.20E-003 | 2.39E-003 | 28.4 | 53.9 | 9.27E-003 |
| Untreated base tress | 2.88 | 1.26E-003 | 2.53E-003 | 27.4 | 52 | 8.90E-003 |

These results show that when compared to a water treatment, betaine has enhanced the elasticity of the hair by penetrating into it. The figures for the work at both 15% and 25% take account of the stress and strain, and are also affected by the steepness of the elastic gradient. The total work is also numerically higher after the betaine treatment than the water.

The results (mean values) for the Oriental hair with the treatment not rinsed from hair are presented in Tables 5a and 5b.

**Table 5a**

| Tress | Elastic modulus MPa | Elastic gradient gmf/mm | Plateau stress (gmf/sq micron) | Stress at 15% (gmf/sq micron) | Stress at 25% (gmf/sq micron) | Break stress (gmf/sq micron) |
|---|---|---|---|---|---|---|
| Treated with betaine | 1.730 | 24.3 | 8.78E-003 | 8.88E-003 | 1.12E-002 | 2.56E-002 |
| Treated with water | 2.060 | 27.4 | 9.84E-003 | 1.03E-002 | 1.30E-002 | 2.62E-002 |
| Untreated base tress | 1.920 | 25.3 | 9.16E-003 | 9.49E-003 | 1.18E-002 | 2.48E-002 |

**Table 5b**

| Tress | Elastic extension (%) | Work @ 15% (joules) | Work @ 25% (joules) | Yield extension (%) | Break extension (%) | Total work (joules) |
|---|---|---|---|---|---|---|
| Treated with betaine | 2.99 | 1.19E-003 | 2.45E-003 | 27.6 | 52.2 | 8.63E-003 |
| Treated with water | 3.03 | 1.29E-003 | 2.69E-003 | 25.9 | 48.9 | 8.33E-003 |
| Untreated base tress | 3.12 | 1.19E-003 | 2.4E-003 | 26.1 | 49.3 | 7.56E-003 |

These results show that the yield and break extension figures are both higher for the betaine treated hair, but the elastic modulus and stress figures are lower. Higher yield extension and break extension show that the hair has extended further before breaking and is therefore more elastic. In particular, the combination of lower elastic modulus and higher break extension indicate that the hair has been treated with a material which has had both a conditioning and a moisturising effect. Break extension is affected by the moisture content of the hair, the brittleness, and the presence of flaws on the hair surface. The betaine treatment appears to have prevented the flaws present on the surface of the hair from propagating and causing fibre fatique until the hair has undergone a longer period of elongation. The low figures for the tress parameters again indicate that the hair has stretched further and these, coupled with the higher break figure, show that the hair is more elastic than the water treated control for the load applied.

### Example 3

A leave-on conditioner product, incorporating betaine in the form of monohydrate was developed. During development of the conditioner, care was taken to restrict the extent of other components in the formulation, so that properties beneficial to the hair can genuinely be attributed to use of the betaine material, rather that to a plethora of other materials.

| Product formulation for a leave-on conditioner | |
|---|---|
| Ingredient | % w/w |
| Polyacrylamide, C13-14 isoparaffin, laureth 7 Sepigel 305 (Seppic UK) | 3.0 |
| D-Panthenol (Roche) | 0.5 |
| Cyclomethicone, Dimethiconol Dow 1401 (Dow Corning) | 5.0 |
| Glycerine (Croda Chemicals) | 3.0 |
| Phenoxyethanol, methyldibromoglutaronitrile, 2-bromo-2-nitropropane-1,3-diol, butyl paraben, isobutyl paraben Nipaguard TBK (Nipa Laboratories) | 0.1 |
| Perfume Organo (Fragrance Oils) | 0.2 |
| Hydrolysed wheat protein hydroxypropyl polysiloxane Crodasone W (Croda) | 1.0 |
| Betaine | 2.0 |
| Deionised water | balance to 100% |

Conditioner was applied to wet hair, which was first washed with a shampoo, rinsed and towel dried. The amount used varying according to the hair length and texture was either 0.75 g, 1.5 g or 2.25 g of product. The conditioner extended the gloss and brightness of the hair compared to a control formulation without betaine. In addition, it left the hair with more body than the control.

## Claims

1. Use of betaine to enhance and/or maintain the elasticity of chemically treated hair.

2. The use according to claim 1, wherein the hair is bleached.

3. The use according to claim 2, wherein the hair is dyed.

4. The use according to any one of claims 1 to 3, wherein the hair is dark hair.

5. The use according to any one of claims 1 to 3, wherein the hair is European type of hair.

6. The use according to any one of claims 1 to 3, wherein the hair is Oriental hair.

7. The use according to any one of claims 1 to 3, wherein the hair is Ethnic hair.

8. The use according to any one of claims 1 to 7 wherein betaine is in the form of a monohydrate, an anhydride or a salt.

9. The use according to any one of claims 1 to 8, wherein betaine is applied to the hair without subsequent rinsing.

10. The use according to claim 9, wherein betaine is formulated into a leave-on formulation.

11. A method of enhancing and/or maintaining elasticity of chemically treated hair comprising applying an effective amount of betaine into the hair.

12. The method according to claim 11, wherein the hair is bleached.

13. The method according to claim 11, wherein the hair is dyed.

14. The method according to any one of claims 11 to 13, wherein the hair is dark hair.

15. The method according to any one of claims 11 to 13, wherein the hair is European type of hair

16. The method according to any one of claims 11 to 13, wherein the hair is Oriental hair.

17. The method according to any one of claims 11 to 13, wherein the hair is Ethnic hair.

18. The method according to any one of claims 11 to 17, wherein betaine is in the form of a monohydrate, an anhydride or a salt.

19. The method according to any one of claims 11 to 18, wherein betaine is applied to the hair without subsequent rinsing.

20. The method according to claim 19, wherein betaine is formulated into a leave-on formulation.

## Patentansprüche

1. Verwendung von Betain zur Erhöhung und/oder zum Erhalt der Elastizität von chemisch behandeltem Haar.

2. Verwendung gemäss Anspruch 1, wobei das Haar gebleicht ist.

3. Verwendung gemäss Anspruch 2, wobei das Haar gefärbt ist.

4. Verwendung gemäss einem der Ansprüche 1 bis 3, wobei das Haar dunkles Haar ist.

5. Verwendung gemäss einem der Ansprüche 1 bis 3, wobei das Haar ein europäischer Haartyp ist.

6. Verwendung gemäss einem der Ansprüche 1 bis 3, wobei das Haar ein orientalischer Haartyp ist.

7. Verwendung gemäss einem der Ansprüche 1 bis 3, wobei das Haar ethnisches Haar ist.

8. Verwendung gemäss einem der Ansprüche 1 bis 7, wobei sich das Betain in Form eines Monohydrats, eines Anhydrids oder eines Salzes befindet.

9. Verwendung gemäss einem der Ansprüche 1 bis 8, wobei das Betain ohne darauffolgende Spülung auf das Haar angewandt wird.

10. Verwendung gemäss Anspruch 9, wobei das Betain in eine Leave-on-Formulierung formuliert wird.

11. Verfahren zur Erhöhung und/oder zum Erhalt der Elastizität von chemisch behandeltem Haar, umfassend die Anwendung einer effektiven Menge an Betain in das Haar.

12. Verfahren gemäss Anspruch 11, wobei das Haar gebleicht ist.

13. Verfahren gemäss Anspruch 11, wobei das Haar gefärbt ist.

14. Verfahren gemäss einem der Ansprüche 11 bis 13, wobei das Haar dunkles Haar ist.

15. Verfahren gemäss einem der Ansprüche 11 bis 13, wobei das Haar ein europäischer Haartyp ist.

16. Verfahren gemäss einem der Ansprüche 11 bis 13, wobei das Haar ein orientalischer Haartyp ist.

17. Verfahren gemäss einem der Ansprüche 11 bis 13, wobei das Haar ethnisches Haar ist.

18. Verfahren gemäss einem der Ansprüche 11 bis 17, wobei sich das Betain in Form eines Monohydrats, eines Anhydrids oder eines Salzes befindet.

19. Verfahren gemäss einem der Ansprüche 11 bis 18, wobei das Betain ohne darauffolgende Spülung auf das Haar angewandt wird.

20. Verfahren gemäss Anspruch 19, wobei das Betain in eine Leave-on-Formulierung formuliert wird.

## Revendications

1. Utilisation de bétaïne pour renforcer et/ou conserver l'élasticité de cheveux traités chimiquement.

2. Utilisation selon la revendication 1, dans laquelle les cheveux sont décolorés.

3. Utilisation selon la revendication 2, dans laquelle les cheveux sont teints.

4. Utilisation selon l'une quelconque des revendications 1 à 3, dans laquelle les cheveux sont des cheveux foncés.

5. Utilisation selon l'une quelconque des revendications 1 à 3, dans laquelle les cheveux sont du type cheveux européens.

6. Utilisation selon l'une quelconque des revendications 1 à 3, dans laquelle les cheveux sont des cheveux orientaux.

7. Utilisation selon l'une quelconque des revendications 1 à 3, dans laquelle les cheveux sont des cheveux ethniques.

8. Utilisation selon l'une quelconque des revendications 1 à 7, dans laquelle la bétaïne est sous la forme d'un monohydrate, d'un anhydride ou d'un sel.

9. Utilisation selon l'une quelconque des revendications 1 à 8, dans laquelle la bétaïne est appliquée sur les cheveux sans rinçage ultérieur.

10. Utilisation selon la revendication 9, dans laquelle la bétaïne est formulée en une formulation que l'on laisse.

11. Procédé de renforcement et/ou de conservation de l'élasticité de cheveux traités chimiquement comprenant l'application d'une quantité efficace de bétaïne sur les cheveux.

12. Procédé selon la revendication 11, dans lequel les cheveux sont décolorés.

13. Procédé selon la revendication 11, dans lequel les cheveux sont teints.

14. Procédé selon l'une quelconque des revendications 11 à 13, dans lequel les cheveux sont des cheveux foncés.

15. Procédé selon l'une quelconque des revendications 11 à 13, dans lequel les cheveux sont du type cheveux européens.

16. Procédé selon l'une quelconque des revendications 11 à 13, dans lequel les cheveux sont des cheveux orientaux.

17. Procédé selon l'une quelconque des revendications 11 à 13, dans lequel les cheveux sont des cheveux ethniques.

18. Procédé selon l'une quelconque des revendications 11 à 17, dans lequel la bétaïne est sous la forme d'un monohydrate, d'un anhydride ou d'un sel.

19. Procédé selon l'une quelconque des revendications 11 à 18, dans lequel la bétaïne est appliquée sur les cheveux sans rinçage ultérieur.

20. Procédé selon la revendication 19, dans lequel la bétaïne est formulée en une formulation que l'on laisse.
